(19) Europäisches Patentamt
European Patent Office
Office européen des brevets

(11) **EP 3 963 314 B1**

(12) **EUROPÄISCHE PATENTSCHRIFT**

(45) Veröffentlichungstag und Bekanntmachung des Hinweises auf die Patenterteilung:
**05.07.2023 Patentblatt 2023/27**

(21) Anmeldenummer: **20712340.7**

(22) Anmeldetag: **18.03.2020**

(51) Internationale Patentklassifikation (IPC):
**G01N 24/08** $^{(2006.01)}$     **G01R 33/44** $^{(2006.01)}$

(52) Gemeinsame Patentklassifikation (CPC):
**G01N 24/082; G01R 33/44**

(86) Internationale Anmeldenummer:
**PCT/EP2020/057434**

(87) Internationale Veröffentlichungsnummer:
**WO 2020/221505 (05.11.2020 Gazette 2020/45)**

(54) **VERFAHREN ZUR ERMITTLUNG EINES TROCKNUNGSVERLAUFS SOWIE FEUCHTEMESSGERÄT**

METHOD FOR DETERMINING A DRYING CURVE, AND MOISTURE METER

PROCÉDÉ POUR LA DÉTERMINATION D'UN PROCESSUS DE SÉCHAGE AINSI QU'APPAREIL DE MESURE D'HUMIDITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.04.2019 DE 102019206181**

(43) Veröffentlichungstag der Anmeldung:
**09.03.2022 Patentblatt 2022/10**

(73) Patentinhaber: **Robert Bosch GmbH 70442 Stuttgart (DE)**

(72) Erfinder: **EDELMANN, Markus 70771 Leinfelden-Echterdingen (DE)**

(56) Entgegenhaltungen:
DE-A1-102014 218 375

- ARENDS THOMAS ET AL: "Moisture transport in pine wood during one-sided heating studied by NMR", EXPERIMENTAL THERMAL AND FLUID SCIENCE, ELSEVIER, AMSTERDAM, NL, Bd. 99, 4. August 2018 (2018-08-04), Seiten 259-271, XP085470941, ISSN: 0894-1777, DOI: 10.1016/J.EXPTHERMFLUSCI.2018.08.004
- PROIETTI NOEMI ET AL: "Evolution of physicochemical properties of pear during drying by conventional techniques, portable-NMR, and modelling", JOURNAL OF FOOD ENGINEERING, BARKING, ESSEX, GB, Bd. 230, 28. Februar 2018 (2018-02-28), Seiten 82-98, XP085363653, ISSN: 0260-8774, DOI: 10.1016/J.JFOODENG.2018.02.028
- COLINART THIBAUT ET AL: "Investigation of Drying of Building Materials by Single-sided NMR", ENERGY PROCEDIA, ELSEVIER, NL, Bd. 78, 30. Dezember 2015 (2015-12-30), Seiten 1484-1489, XP029375794, ISSN: 1876-6102, DOI: 10.1016/J.EGYPRO.2015.11.174

**EP 3 963 314 B1**

**Beschreibung**

Stand der Technik

Die zuverlässige Bestimmung von Material- und Baufeuchte stellt einen wichtigen Aspekt im modernen Bauwesen dar. Für eine zerstörungsfreie Messung einer Feuchtigkeit eines zu untersuchenden Materials vor Ort werden derzeit handelsübliche Verfahren und Feuchtemessgeräte eingesetzt, die auf dielektrischen Messungen basieren. Mittels dieser Verfahren und Feuchtemessgeräte lassen sich aktuelle Feuchtewerte bestimmen oder eine kontinuierliche Ausgabe der Feuchtewerte realisieren.

Ferner sind aus DE 10 2014 218 375 A1 mobile NMR-Messgeräte mit einer Sensorvorrichtung bekannt, wobei die Sensorvorrichtung zumindest einen Kernspinresonanz-Sensor (NMR-Sensor) aufweist, der zur Bestimmung eines Feuchtewerts eines zu untersuchenden Materials vorgesehen ist.

Aus dem Artikel Arends, Thomas et al., "Moisture transport in pine wood during one-sided heating studied by NMR", Eperimental Thermal and Fluid Science, Bd. 99, 4. August 2018, Seiten 259-271, ist ein Verfahren zur Bestimmung des Feuchtigkeitstransports in beheizten Hölzern bekannt.

In dem Artikel Proietti N. et al., "Evolution of physicochemical properties of pear during drying by conventional tehcniques, portable-NMR, and modelling", Journal of Food Engineering, Bd. 230, 28.02.2018, Siten 82-98, wird ein Trocknungsverfahren für Lebensmittel beschrieben.

Die vorliegende Erfindung betrifft ein computerimplementiertes Verfahren zur Ermittlung eines zeitabhängigen Trocknungsverlaufs $\square(t)$ eines Untersuchungsobjekts sowie ein Feuchtemessgerät zur Durchführung des Verfahrens.

> Sitz: Stuttgart, Registergericht: Amtsgericht Stuttgart, HRB 14000;
> Aufsichtsratsvorsitzender: Prof. Dr. Stefan Asenkerschbaumer; Geschäftsführung: Dr. Stefan Hartung,
> Dr. Christian Fischer, Filiz Albrecht, Dr. Markus Forschner, Dr. Markus Heyn, Rolf Naiork

Offenbarung der Erfindung

Es wird ein computerimplementiertes Verfahren zur Ermittlung eines zeitabhängigen Trocknungsverlaufs $\tau(t)$ eines Untersuchungsobjekts vorgeschlagen. Das erfindungsgemäße Verfahren ist durch zumindest folgende Verfahrensschritte gekennzeichnet:

- Bereitstellen von Messdaten betreffend eine zeitabhängige Feuchteverteilung $\phi^i(t)$ bis zu einer Tiefe d' des Untersuchungsobjekts,

- Ermitteln eines zeitabhängigen reduzierten Trocknungsverlaufs $\tau^i(t)$ unter Verwendung der zeitabhängigen Feuchteverteilung $\phi^i(t)$, wobei der zeitabhängige reduzierte Trocknungsverlauf mittels $\tau^i(t) = \int_0^{d^i} \Phi^i(x,t)\, dx$ berechnet wird, alternativ durch Mitteln dieses Integrals über $d^i$, wobei die Tiefe $d^i$ kleiner ist als eine Gesamtdicke des Untersuchungsobjekts (10),
- Kalibrieren eines das Untersuchungsobjekt betreffenden Materialmodells unter Verwendung des zeitabhängigen reduzierten Trocknungsverlaufs $\tau^i(t)$,
- Ermitteln, insbesondere Simulieren, eines zeitabhängigen Trocknungsverlaufs $\tau(t)$ des Untersuchungsobjekts für dessen Gesamtdicke unter Verwendung des kalibrierten Materialmodells, wobei aus dem zeitabhängigen Trocknungsverlauf $\tau(t)$ des Untersuchungsobjekts ein Trocknungszeitpunkt ermittelt wird, insbesondere extrapoliert wird, für den ein Wert des zeitabhängigen Trocknungsverlaufs unter eine Schwelle fällt.

Unter "ermitteln eines zeitabhängigen Trocknungsverlaufs $\tau(t)$ eines Untersuchungsobjekts" ist zu verstehen, dass ein Zusammenhang oder eine Zuordnungsvorschrift, beispielsweise eine Funktion, eine Kalibriervorschrift, eine Tabelle oder dergleichen, ermittelt wird, die ermöglicht, zu einem gegebenen Zeitpunkt t einen absoluten oder relativen Feuchtewert und/oder einen absoluten oder relativen Trocknungswert anzugeben. Die Begriffe Feuchtigkeit und Trocknungsgrad bzw. Feuchteverlauf und Trocknungsverlauf werden in dieser Schrift synonym verwendet. Der Trocknungsgrad beschreibt dabei einen Feuchtegehalt des Untersuchungsobjekts im Falle eines Trocknungsprozesses. Analog bezeichnet der Trocknungsverlauf einen Feuchteverlauf während eines Trocknungsprozesses. Ein Feuchtewert spezifiziert die im Untersuchungsobjekt noch vorhandene Feuchtigkeit und kann beispielsweise durch eine Konzentration verdampfbaren Wassers in dem Untersuchungsobjekt (zu unterscheiden von chemisch fest gebundenem Kristallwasser oder Hydratwasser) angegeben werden. Insbesondere ermöglicht es der Zusammenhang oder die Zuordnungsvorschrift des zeitabhängigen Trocknungsverlaufs $\tau(t)$, eine Vorhersage für Zeitpunkte t auch in der Zukunft anzugeben, insbesondere zu extrapolieren. Der zeitabhängige Trocknungsverlauf $\tau(t)$, der messtechnisch typischerweise nicht unmittelbar zugänglich ist, wird erfindungsgemäß aus bereitgestellten Messdaten für das zu Grunde liegende Untersuchungsobjekt ermittelt, insbesondere simuliert.

Unter einem "Untersuchungsobjekt" soll ein Material verstanden werden, das auf eine in dem Material enthaltene Feuchtigkeit bzw. auf eine in dem Material nicht mehr enthaltenen Feuchtigkeit, d.h. einen Trocknungsgrad, untersucht werden soll. Beispielsweise und nicht abschließend kann es sich dabei um eine Wand, einen Boden, eine Decke, Estrich, ein organisches Gebilde (insbesondere Teile eines Körpers) und/oder ein Teil eines Geländes handeln. Bestehen kann ein Untersuchungsobjekt beispielsweise aus Holz, Glas, Kunststoff, Beton, Estrich, Stein, Ziegel, Gips, Metall, organischen Materialien oder dergleichen.

Unter "computerimplementiert" ist insbesondere zu verstehen, dass das Verfahren in Form von Software oder in einer Mischung aus Software und Hardware realisiert ist. Das Verfahren wird dabei mittels einer Prozessorvorrichtung, insbesondere einer Steuervorrichtung und/oder einer Auswertevorrichtung, ganz insbesondere einer Steuervorrichtung und/oder einer Auswertevorrichtung eines Feuchtemessgeräts, durchgeführt. Zur Durchführung des Verfahrens kann die Prozessorvorrichtung, insbesondere die Steuervorrichtung und/oder die Auswertevorrichtung, ferner zumindest eine Speichervorrichtung aufweisen, in der das Verfahren als maschinenlesbares Computerprogramm hinterlegt ist.

Unter "Bereitstellen von Messdaten" ist insbesondere zu verstehen, dass der das Verfahren durchführenden Vorrichtung, insbesondere einer Prozessorvorrichtung, einer Steuervorrichtung, einer Auswertevorrichtung oder dergleichen, die Messdaten zur weiteren Verarbeitung zur Verfügung gestellt werden. In einer Ausführungsform des Verfahrens kann die entsprechende Information durch einen Bediener bereitgestellt werden. Das Bereitstellen kann dabei insbesondere durch eine Eingabe oder Auswahl durch einen Bediener, beispielsweise mittels einer Eingabevorrichtung oder mittels einer Menüauswahl oder dergleichen, erfolgen. In einer alternativen oder zusätzlichen Ausführungsform des Verfahrens kann das Bereitstellen durch das Einlesen der Messdaten aus einer Datei, beispielsweise durch Einlesen von auf einem Datenserver hinterlegten Daten, erfolgen. In einer wiederum alternativen oder zusätzlichen Ausführungsform des Verfahrens können die Messdaten unter Verwendung einer Sensorvorrichtung, insbesondere einer Sensorvorrichtung des das Verfahren ausführenden Feuchtemessgeräts, ermittelt oder gemessen und anschließend bereitgestellt werden. Eine Eingabevorrichtung stellt dabei typischerweise ebenfalls eine entsprechende Sensorvorrichtung dar. Unter der Sensorvorrichtung ist eine Vorrichtung zu verstehen, mittels der entsprechende Messdaten von dem Untersuchungsobjekt erfasst werden können. Eine derartige Sensorvorrichtung kann insbesondere einen oder mehrere Sensoren aufweisen. In einem Ausführungsbeispiel kann die Sensorvorrichtung als ein tiefenauflösender Feuchtesensor, insbesondere ein tiefenauflösender NMR-Sensor, ein tiefenauflösendes Feuchtesensorarray (umfassend eine Mehrzahl von in das Untersuchungsobjekt eingebetteten Feuchtesensoren), ein tiefenauflösendes Radar oder dergleichen oder als eine Kombination derer realisiert sein. Weitere tiefenauflösende Feuchtesensoren sind dem Fachmann bekannt. Durch das Bereitstellen von Messdaten mittels zumindest einer Sensorvorrichtung kann eine besonders präzise und zuverlässige Bestimmung dieser Messdaten ermöglicht werden. Insbesondere können derart an ein Untersuchungsobjekt angepasste Messdaten zur Durchführung des Verfahrens bereitgestellt werden. Ferner können Fehleingaben, insbesondere durch Abweichungen von tatsächlich vorliegenden Messdaten, vermieden werden.

Die bereitgestellten Messdaten betreffen zumindest eine zeitabhängige Feuchteverteilung $\phi^i(t)$ zumindest bis zu einer Tiefe d' des Untersuchungsobjekts. Die Feuchteverteilung $\phi^i(t)$ kann insbesondere als zeitabhängige, vertikale Feuchteverteilung bzw. als Feuchte-Tiefenprofil in dem Untersuchungsobjekt - d.h. in das Untersuchungsobjekt hinein - ermittelt und bereitgestellt werden. Derartige Messdaten können insbesondere unter Verwendung der oben genannten tiefenauflösenden Feuchtesensoren gemessen und somit ermittelt und bereitgestellt werden. Vorteilhaft kann derart ein vereinfachtes Verfahren angegeben werden, bei dem keine das Untersuchungsobjekt vollständig durchdringende Feuchteverteilung (d.h. bis zur maximalen Tiefe, die der Stärke des Untersuchungsobjekts entspricht) ermittelt werden muss. Die Anforderung an eine Rechenleistung der zur Durchführung des Verfahrens verwendeten Prozessorvorrichtung und/oder an einen zu verwendenden Feuchtesensor kann somit erheblich reduziert werden.

Unter Verwendung der zeitabhängigen Feuchteverteilung $\phi^i(t)$ wird anschließend zumindest ein zeitabhängiger reduzierter Trocknungsverlauf $\tau^i(t)$, insbesondere zumindest bis zu der Tiefe $d^i$, des Untersuchungsobjekts ermittelt. In einem Ausführungsbeispiel kann der zeitabhängige reduzierte Trocknungsverlauf $\tau^i(t)$ durch Integrieren der Feuchteverteilung $\phi^i(t)$ bis zur Tiefe d' berechnet werden:

$$\tau^i(t) = \int_0^{d^i} \Phi^i(x, t)\, dx$$

Alternativ kann der reduzierte Trocknungsverlauf $\tau^i(t)$ durch Mitteln der Feuchteverteilungen $\phi^i(t)$ berechnet werden:

$$\tau^i(t) = \frac{1}{d^i} \int_0^{d^i} \Phi^i(x, t)\, dx$$

Der zeitabhängige Trocknungsverlauf beschreibt dabei die (mittlere) Feuchtigkeit, welche sich während der Trocknung in der Schicht mit Dicke d' befindet. Im Falle, dass die Dicke d' (nicht erfindungsgemäss) der Gesamtdicke des Untersuchungsobjekts ($d^{ges}$) entspricht, ist der zeitabhängige reduzierte Trocknungsverlauf $\tau^i(t)$ identisch mit dem zeitabhängiger Trocknungsverlauf $\tau(t)$.

Der zeitabhängige reduzierte Trocknungsverlauf $\tau^i(t)$ dient anschließend der Kalibrierung eines das Untersuchungsobjekt betreffenden Materialmodells. Das Materialmodell umfasst insbesondere physikalische und/oder chemische Parameter und Effekte, die für den zeitabhängigen Trocknungsverlauf $\tau(t)$ relevant sind. In einer Ausführungsform des erfindungsgemäßen Verfahrens berücksichtigt das Materialmodell zumindest eine Flüssigwasserleitfähigkeit, eine Dampfleitfähigkeit und einen Wasserdampfoberflächenaustausch des Untersuchungsobjekts. Unter "Kalibrierung" des Materialmodells ist zu verstehen, dieses mit Initialwerten derart zu konfigurieren, dass es das tatsächlich zu charakterisierende Untersuchungsobjekt möglichst realitätsgetreu beschreibt. Die Kalibrierung erfolgt insbesondere durch Anpassen des Materialmodells derart, dass es den zumindest einen zeitabhängigen reduzierten Trocknungsverlauf $\tau^i(t)$ möglichst gut beschreibt - beispielsweise durch Anfitten des Materialmodells an den zumindest einen zeitabhängigen reduzierten Trocknungsverlauf $\tau^i(t)$.

Erfindungsgemäß kann derart ein Verfahren angegeben werden, das der Bestimmung bzw. Kalibrierung von in dem Materialmodell eingehenden Materialeigenschaften - insbesondere Wassertransporteigenschaften - auf Basis von im Vorfeld bereitgestellten, insbesondere gemessenen, Messdaten zu einem Untersuchungsobjekt dient. Insbesondere erlaubt es das Materialmodell, trocknungsrelevante Materialeigenschaften zu berücksichtigen, mittels der sich das Untersuchungsobjekt hinsichtlich seiner Trocknungseigenschaften besonders gut charakterisieren und beschreiben lässt. Das Materialmodell beschreibt insbesondere zumindest Feuchtetransportphänomene in dem Untersuchungsobjekt. In einem Ausführungsbeispiel kann das Materialmodell als ein sogenanntes Porenmodel realisiert sein, wie es für kapillarporöse Materialen bekannt ist, vergleiche beispielsweise das aus dem Stand der Technik bekannte Modell von Burdine (Burdine, 1953, "Relative Permeability Calculations From Pore Size Distribution Data") oder Mualem (Mualem, 1976, "A new model for predicting the hydraulic conductivity of unsaturated porous media"). Insbesondere kann das Materialmodell einen sogenannten Kontinuum-Ansatz umfassen, in dem die den Feuchtetransportprozessen zu Grunde liegenden physikalischen Effekte durch Transportgleichungen beschreibbar sind (vergleiche: "Coupled heat air and moisture transfer in building structures", P. Häupl, J. Grunewald, H. Fechner, H. Stopp). Mittels der Kalibrierung des Materialmodells kann das Materialmodell an die Materialeigenschaften des Untersuchungsobjekts angepasst werden und erlaubt somit, unter Anwendung des Materialmodells besonders präzise Aussagen zu treffen.

Abschließend wird ein zeitabhängiger Trocknungsverlauf $\tau(t)$ des gesamten Untersuchungsobjekts unter Verwendung des kalibrierten Materialmodells ermittelt, insbesondere simuliert. Dabei beruht der derart ermittelte zeitabhängige Trocknungsverlauf $\tau(t)$ auf Informationen über die Feuchtetransporteigenschaften des Untersuchungsobjekts (einschließlich Randbedingungen wie Oberflächengröße oder dergleichen). Ausgehend vom zeitabhängigen reduzierten Trocknungsverlauf $\tau^i(t)$ kann somit auf die Eigenschaften des gesamten Untersuchungsobjekts geschlossen werden. Insbesondere ist es mittels des nunmehr ermittelten zeitabhängigen Trocknungsverlaufs $\tau(t)$ möglich, eine Aussage zur zeitabhängigen Feuchtigkeit bzw. zeitabhängigen Trocknungsgrad des gesamten Untersuchungsobjekts zu treffen - basierend auf den vorab bereitgestellten Messdaten, die letztlich einer Information über lediglich einen Teil des Untersuchungsobjekts entsprechen.

Das erfindungsgemäße Verfahren ermöglicht es, besonders zügig eine Kalibrierung eines ein Untersuchungsobjekt beschreibenden Materialmodells durchzuführen, unter dessen Verwendung ein Feuchtetransport in dem Untersuchungsobjekt für verschiedene Zeitpunkte auswertbar, insbesondere berechenbar, ganz insbesondere extrapolierbar, ist. Das kalibrierte Materialmodell erlaubt, Aussagen zu einer Feuchtigkeit bzw. zu einem Trocknungsgrad des gesamten Untersuchungsobjekts zu treffen. Ferner erlaubt das Materialmodell, einschließlich der Charakterisierung der Wassertransporteigenschaften des Untersuchungsobjekts, Aussagen zu verschiedenen Aspekten zu treffen, beispielsweise zu einer energetischen Bewertung und/oder einem Schimmelrisiko des Untersuchungsobjekts. Gegenüber aus dem Stand der Technik bekannten Verfahren, bei denen die Charakterisierung des Untersuchungsobjekts sowie die Kalibrierung des Materialmodells eine einige Monate umfassende Aufgabe darstellt, kann unter Anwendung des erfindungsgemäßen Verfahrens eine Aussage über einen zeitabhängigen Trocknungsverlauf in deutlich geringerer Zeitspanne erhalten werden.

In einer Ausführungsform des erfindungsgemäßen Verfahrens wird aus dem zeitabhängigen Trocknungsverlauf $\tau(t)$ des Untersuchungsobjekts ein Trocknungszeitpunkt $t_0$ ermittelt, insbesondere extrapoliert. Für den Trocknungszeitpunkt $t_0$ gilt, dass ein Wert $\tau(t_0)$ des zeitabhängigen Trocknungsverlaufs unter eine definierte, insbesondere vorgebbare, Schwelle $\tau_0$ fällt. Auf diese Weise kann auf zuverlässige Weise abgeschätzt werden, wann das Untersuchungsobjekt als "trocken" betrachtet werden kann, insbesondere bezogen auf einen erforderlichen Trocknungsgrad des Untersuchungsobjekts bei einer nachfolgenden Behandlung des Untersuchungsobjekts. Insbesondere bei Aufgaben im Baugewerbe, wie beispielsweise bei der Weiterverarbeitung von gegossenem Beton oder Estrich, ist die frühzeitige Bestimmung und Kenntnis dieses Zeitpunktes besonders vorteilhaft.

In einer Ausführungsform des erfindungsgemäßen Verfahrens werden im letzten Verfahrensschritt und/oder in einem weiteren Verfahrensschritt den zeitabhängigen Trocknungsverlauf $\tau(t)$ des Untersuchungsobjekts beeinflussende Trocknungsparameter modelliert bzw. simuliert und/oder variiert. So können insbesondere Trocknungsparameter wie Temperatur, Wärmezufuhr, Luftaustauschrate, Luftfeuchtigkeit, Strahlungseinfluss (z.B. Sonneneinstrahlungsintensität, Sonneneinstrahlungsdauer) und dergleichen in dem Materialmodell umfasst und somit prinzipiell berücksichtigbar sein. Die Trocknungsparameter betreffen insbesondere Rahmenbedingungen, unter denen die Trocknung des Untersuchungsobjekts erfolgt. In einer Ausführungsform des erfindungsgemäßen Verfahrens werden optimierte Trocknungsparameter, die in der Modellierung bzw. Simulation zu einem schnelleren zeitabhängigen Trocknungsverlauf $\tau^{opt}(t)$ des Untersuchungsobjekts führen, berechnet. Unter "schneller" ist hier insbesondere "verkürzt" bzw. "beschleunigt" zu verstehen. Diese Berechnung kann beispielsweise durch Variieren der Trocknungsparameter erfolgen, bei gleichzeitiger Minimierung des zeitabhängigen Trocknungsverlaufs $\tau^{opt}(t)$ oder Minimierung von $t_0$ (Zeit bei der der Grenzwert $\tau_0$ erreicht wird). Ist ein derartiges Minimum in dem zeitabhängigen Trocknungsverlauf $\tau^{opt}(t)$ gefunden, können die optimierten Trocknungsparameter in einer Ausführungsform des erfindungsgemäßen Verfahrens als Handlungsanweisung an einen Nutzer des Verfahrens ausgegeben werden. Unter Handlungsanweisung ist hier beispielsweise ein Vorschlag zu verstehen, wie der Nutzer die Trocknungsparameter verändern könnte, um zu einem beschleunigten Trocknungsverlauf des Untersuchungsobjekts zu gelangen. Derartige Handlungsanweisungen könnten beispielsweise Anweisungen wie "Lüften" oder "Fenster öffnen" oder "Temperatur erhöhen" oder dergleichen umfassen. Derart kann ein Nutzer des Verfahrens die Bedingungen, unter denen die Trocknung des Untersuchungsobjekts stattfindet, gezielt derart beeinflussen, dass eine möglichst zügige Trocknung des Untersuchungsobjekts realisiert wird.

In einer Ausführungsform des erfindungsgemäßen Verfahrens werden die Messdaten betreffend die zumindest eine zeitabhängige Feuchteverteilung $\phi^i(t)$ für mindestens sechs verschiedene Zeitpunkte, insbesondere für mindestens sechs verschiedene Tage, bereitgestellt. Auf diese Weise kann unter Verwendung von Messdaten, die die zeitabhängige Feuchteverteilung $\phi^i(t)$ zu einem ersten Zeitpunkt betreffen, das Materialmodell zunächst initial (und grob) kalibriert werden. Die weiteren Messdaten, die die zeitabhängige Feuchteverteilung $\phi^i(t)$ zu weiteren Zeitpunkten betreffen, erlauben es anschließend, die Kalibrierung des Materialmodells, insbesondere parallel zum Trocknungsprozess des Untersuchungsobjekts, zu rekalibrieren, insbesondere zu verfeinern, bis eine besonders hohe Übereinstimmung von Materialmodell und Untersuchungsobjekt vorliegt. Auf diese Weise kann eine unter Verwendung des Materialmodells erhaltene Aussage weiter konkretisiert und insbesondere genauer getroffen werden. In einer Ausführungsform des erfindungsgemäßen Verfahrens wird auch die Ermittlung des zeitabhängigen Trocknungsverlaufs $\tau(t)$ des Untersuchungsobjekts unter Verwendung weiterer Messdaten verfeinert, insbesondere kontinuierlich oder quasikontinuierlich (d.h. wiederholt) verfeinert.

In einer Ausführungsform des erfindungsgemäßen Verfahrens werden die Messdaten betreffend die zumindest eine zeitabhängige Feuchteverteilung $\phi^i(t)$ für mindestens zwei, insbesondere für mindestens drei, Tiefen d' des Untersuchungsobjekts bereitgestellt. Die Verwendung einer Mehrzahl von Feuchteverteilungen $\phi^i(t)$, die sich hinsichtlich der Tiefen d' unterscheiden, bis zu der die Feuchteverteilung $\phi^i(t)$ gilt, erlaubt es vorteilhaft, mehrdeutige Lösungen bei der Kalibrierung des Materialmodells, d.h. bei der numerischen Anpassung der dem Materialmodell zu Grunde liegenden Gleichungssysteme, zu vermeiden und somit das Materialmodell robuster zu machen. In einem Ausführungsbeispiel werden Messdaten betreffend die zumindest eine zeitabhängige Feuchteverteilung $\phi^i(t)$ für Tiefen bis zu 1 cm, 1.5 cm, 2 cm, 2.5 cm, 3 cm, 3.5 cm und 4 cm unter Verwendung eines tiefenaufgelösten Feuchtesensors, insbesondere eines NMR-Sensors, gemessen und bereitgestellt.

In einer Ausführungsform des erfindungsgemäßen Verfahrens, werden die Messdaten unter Verwendung eines tiefenauflösenden Feuchtesensors, insbesondere eines tiefenauflösenden NMR-Sensors und/oder eines tiefenauflösenden Feuchtesensorarrays und/oder eines tiefenauflösenden Radars, gemessen und bereitgestellt.

Ferner wird ein Feuchtemessgerät mit zumindest einem tiefenauflösenden Feuchtesensor und mit zumindest einer Steuervorrichtung, insbesondere zur Steuerung des Feuchtemessgeräts, vorgeschlagen, wobei die Steuervorrichtung ferner zur Durchführung des erfindungsgemäßen Verfahrens eingerichtet ist. Insbesondere umfasst das Feuchtemessgerät ferner einen tiefenauflösenden Feuchtesensor, insbesondere einen tiefenauflösenden NMR-Sensor und/oder ein tiefenauflösenden Feuchtesensorarray und/oder ein tiefenauflösendes Radar zur Messung und Bereitstellung der Messdaten. In einer Ausführungsform des Feuchtemessgeräts ist dieses als ein mobiles Feuchtemessgerät, insbesondere als ein Handmessgerät, realisiert. Unter der Steuervorrichtung soll insbesondere eine Vorrichtung mit zumindest einer Steuerelektronik verstanden werden, die Mittel zur Kommunikation mit anderen Komponenten des Messgeräts, beispielsweise Mittel zur Steuerung und Regelung eines Feuchtesensors, und/oder Mittel zur Datenverarbeitung aufweist. Insbesondere ist die Steuervorrichtung dazu vorgesehen, zumindest einen Betriebsfunktionsparameter des Messgeräts einzustellen. Unter "vorgesehen" soll insbesondere speziell "programmiert", "ausgelegt" und/oder "ausgestattet" verstanden werden. Darunter, dass ein Objekt zu einer bestimmten Funktion "vorgesehen" ist, soll insbesondere verstanden werden, dass das Objekt diese bestimmte Funktion in zumindest einem Anwendungs- und/oder Betriebszustand erfüllt und/oder ausführt oder dazu ausgelegt ist, die Funktion zu erfüllen. Vorteilhaft kann unter der Steuerelektronik der erfindungsgemäßen Steuervorrichtung eine Prozessoreinheit in Verbindung mit einer Speichereinheit sowie mit einem in der Speichereinheit gespeicherten Betriebsprogramm verstanden werden, das während des Steuervorgangs ausgeführt wird. Insbesondere können die elektronischen Bauteile der Steuervorrichtung auf einer Platine (Leiterplatte) angeordnet sein, bevorzugt in Form eines Mikrokontrollers. Insbesondere kann die Steuervorrichtung darüber hinaus dazu vorgesehen sein, das gesamte Messgerät zu steuern und dessen Betrieb zu ermöglichen. Des Weiteren weist die Steuervorrichtung gespeicherten Programmcode in Form eines Computerprogramms auf, dessen Ausführung es erlaubt, das erfindungsgemäße Verfahren auszuführen.

In einem weiteren Aspekt wird ein Computerprogramm vorgeschlagen. Das Computerprogramm ist eingerichtet, das vorherig genannte Verfahren auszuführen. Das Computerprogramm umfasst Anweisungen, die eine Prozessorvorrichtung veranlassen, das genannte Verfahren mit all seinen Schritten auszuführen.

Ferner wird ein computerlesbares Speichermedium vorgeschlagen, auf welchem das Computerprogramm hinterlegt, insbesondere gespeichert, ist. Speichermedien an sich sind einem Fachmann dabei bekannt.

Zeichnungen

Die Erfindung ist anhand von in den Zeichnungen dargestellten Ausführungsbeispielen in der nachfolgenden Beschreibung näher erläutert. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen. Gleiche oder ähnliche Bezugszeichen in den Figuren bezeichnen gleiche oder ähnliche Elemente.

Es zeigen:

Figur 1   ein Verfahrensdiagramm einer Ausführungsform des erfindungsgemäßen Verfahrens zur Ermittlung eines zeitabhängigen Trocknungsverlaufs $\tau(t)$ eines Untersuchungsobjekts,

Figur 2   eine schematische Perspektivansicht eines beispielhaften Messszenarios unter Verwendung eines erfindungsgemäßen Feuchtemessgeräts,

Figur 3   für das in Figur 2 dargestellte Messszenario ermittelbare Kurven eines zeitabhängigen reduzierten Trocknungsverlaufs $\tau^i(t)$ und eines zeitabhängigen reduzierten Trocknungsverlaufs $\tau(t)$.

Beschreibung der Ausführungsbeispiele

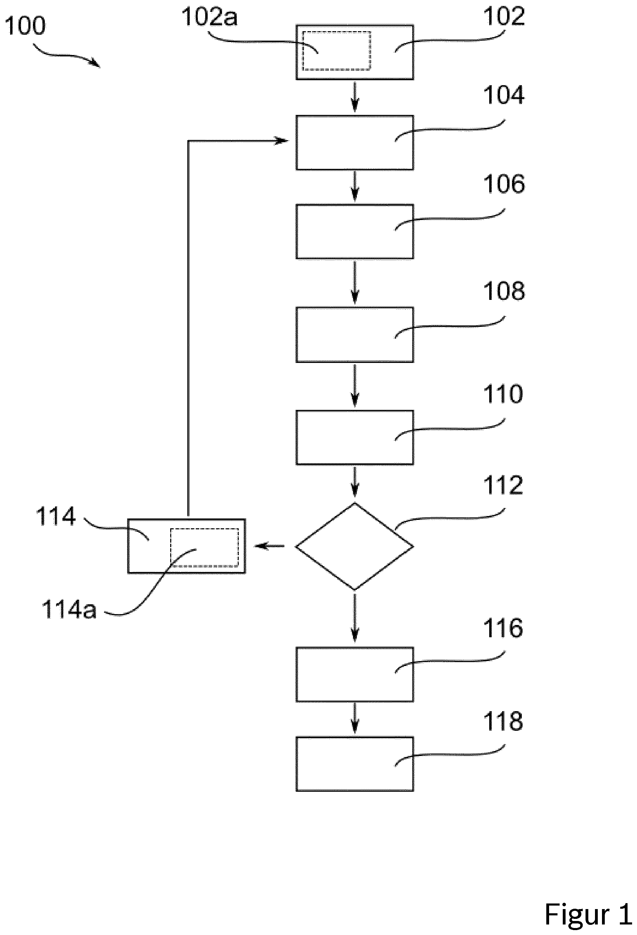

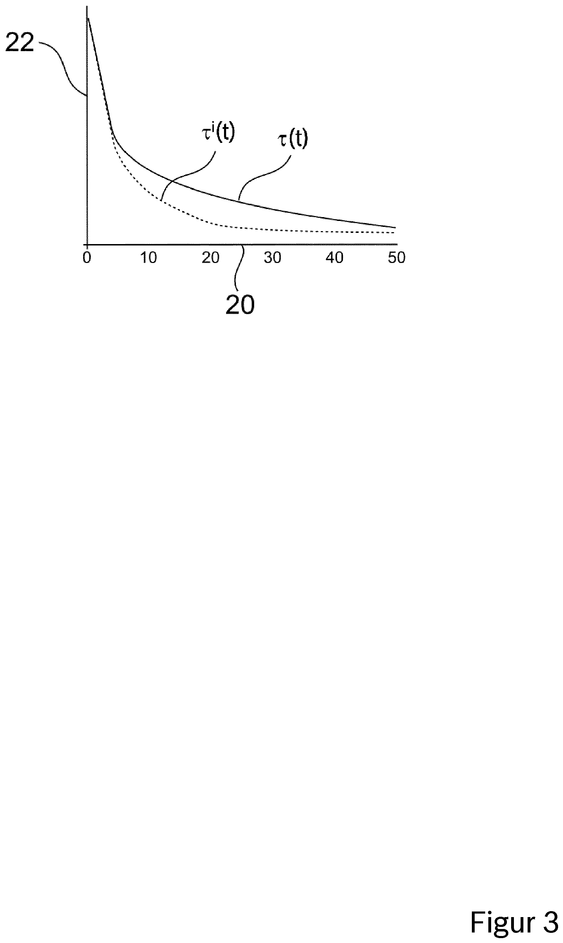

In Figur 1 ist ein Verfahrensdiagramm 100 gezeigt, dass ein Ausführungsbeispiel des erfindungsgemäßen computerimplementierten Verfahrens zur Ermittlung eines zeitabhängigen Trocknungsverlaufs $\tau(t)$ eines Untersuchungsobjekts 10 wiedergibt, wobei der zeitabhängigen Trocknungsverlaufs $\tau(t)$ einen funktionellen Zusammenhang zwischen Zeit und Trocknungsgrad (bzw. Feuchtigkeit) des Untersuchungsobjekts 10 spezifiziert (vgl. Figur 3).

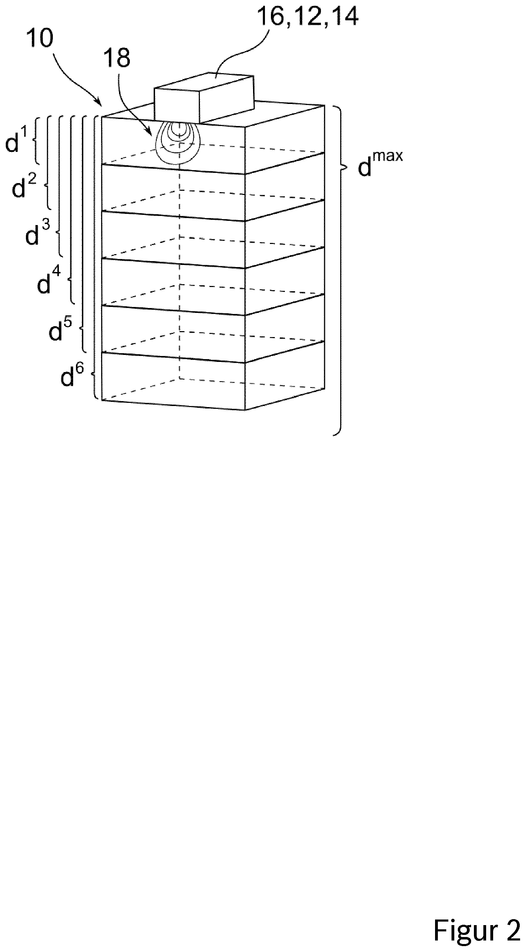

In Verfahrensschritt 102 werden Messdaten betreffend zumindest eine zeitabhängige Feuchteverteilung $\phi^i(t)$ zumindest bis zu einer Tiefe d' (vgl. auch Figur 2) des Untersuchungsobjekts 10 bereitgestellt, insbesondere einer das Verfahren ausführenden Prozessorvorrichtung bereitgestellt. In dem dargestellten Ausführungsbeispiel des Verfahrens werden Messdaten betreffend sechs zeitabhängige Feuchteverteilung $\phi^1(t)$, $\phi^2(t)$, $\phi^3(t)$, $\phi^4(t)$, $\phi^5(t)$, $\phi^6(t)$ bereitgestellt, wobei die zeitabhängigen Feuchteverteilungen entsprechend jeweils bis zu sechs unterschiedlichen Tiefen $d^1$, $d^2$, $d^3$, $d^4$, $d^5$, $d^6$ reichen. Diese sechs Tiefen können beispielsweise bis 1 cm, bis 1.5 cm, bis 2 cm, bis 2.5 cm, bis 3 cm und bis 3.5 cm betragen (vgl. Figur 2). Die Messdaten können optional in Verfahrensschritt 102a gemessen und dann der Prozessorvorrichtung bereitgestellt werden. Beispielsweise können die Messdaten unter Verwendung eines tiefenauflösenden Feuchtesensors 12, insbesondere eines tiefenauflösenden NMR-Sensors 14, eines Feuchtemessgeräts 16 erfasst und bereitgestellt werden, wie dies in Figur 2 schematisch dargestellt ist.

In Verfahrensschritt 104 werden die bereitgestellten Messdaten verarbeitet, insbesondere zur Auswertung vorbereitet, beispielsweise durch Glätten, Filtern oder dergleichen. Ferner wird aus den derart vorbereiteten Messdaten zumindest ein zeitabhängiger reduzierter Trocknungsverlauf $\tau^i(t)$ des Untersuchungsobjekts 10 ermittelt, insbesondere berechnet. Insbesondere werden in diesem Ausführungsbeispiel sechs zeitabhängige reduzierte Trocknungsverläufe $\tau^1(t)$, $\tau^2(t)$, $\tau^3(t)$, $\tau^4(t)$, $\tau^5(t)$, $\tau^6(t)$ unter Verwendung der sechs zeitabhängigen Feuchteverteilungen $\phi^1(t)$, $\phi^2(t)$, $\phi^3(t)$, $\phi^4(t)$, $\phi^5(t)$, $\phi^6(t)$ ermittelt. Ein jeweiliger zeitabhängiger reduzierter Trocknungsverlauf $\tau^i(t)$ wird in diesem Ausführungsbeispiel durch Integrieren der entsprechenden Feuchteverteilung $\phi^i(t)$ bis zur entsprechenden Tiefe d' berechnet:

$$\tau^i(t) = \frac{1}{d^i} \int_0^{d^i} \Phi^i(x,t)\, dx.$$

Ein Beispiel eines zeitabhängigen reduzierten Trocknungsverlaufs $\tau^i(t)$ ist in Figur 3 dargestellt.

In Verfahrensschritt 106 wird ein das Untersuchungsobjekt 10 betreffendes Materialmodell unter Verwendung des zumindest einen zeitabhängigen reduzierten Trocknungsverlaufs $\tau^i(t)$ kalibriert, in diesem Ausführungsbeispiel also unter Verwendung der sechs zeitabhängigen reduzierten Trocknungsverläufe $\tau^1(t)$, $\tau^2(t)$, $\tau^3(t)$, $\tau^4(t)$, $\tau^5(t)$, $\tau^6(t)$. Das Materialmodell umfasst dabei physikalische und/oder chemische Parameter und Effekte, die für einen zeitabhängigen Trocknungsverlauf $\tau(t)$ des gesamten Untersuchungsobjekts 10 relevant sind. Das Materialmodell beschreibt zumindest Feuchtetransportphänomene in dem Untersuchungsobjekt 10 und berücksichtigt dabei zumindest eine Flüssigwasserleitfähigkeit, eine Dampfleitfähigkeit und einen Wasserdampfoberflächenaustausch des Untersuchungsobjekts 10. In diesem Ausführungsbeispiel basiert das Materialmodell auf aus dem Stand der Technik bekannten Modellen wie beispielsweise dem DELPHIN-Modell (vergleiche: "Coupled heat air and moisture transfer in building structures", P. Häupl, J. Grunewald, H. Fechner, H. Stopp). Das Materialmodell wird durch Anfitten an die sechs zeitabhängigen reduzierten Trocknungsverläufe $\tau^1(t)$, $\tau^2(t)$, $\tau^3(t)$, $\tau^4(t)$, $\tau^5(t)$, $\tau^6(t)$ initial kalibriert, wobei Fit-Parameter optimiert (beispielsweise unter Anwendung der Methode der kleinsten Quadrate (leastsquare-fit)) und somit ein das Untersuchungsobjekt 10 möglichst gut beschreibendes Materialmodell erhalten wird.

In Verfahrensschritt 108 wird ein zeitabhängiger Trocknungsverlauf $\tau(t)$ des Untersuchungsobjekts 10 unter Verwendung des kalibrierten Materialmodells ermittelt, insbesondere simuliert. Aus dem Materialmodell sowie aus dem simulierten zeitabhängigen Trocknungsverlauf $\tau(t)$ lassen sich anschließend in Verfahrensschritt 110 unterschiedliche Aussagen durch Auswerten des Materialmodells und/oder des zeitabhängigen Trocknungsverlaufs $\tau(t)$ ableiten. Insbesondere ist es möglich, eine Aussage zur zeitabhängigen Feuchtigkeit bzw. zum zeitabhängigen Trocknungsgrad des gesamten Untersuchungsobjekts 10 zu treffen. Insbesondere wird in Verfahrensschritt 110 aus dem zeitabhängigen Trocknungsverlauf $\tau(t)$ des Untersuchungsobjekts 10 ein Trocknungszeitpunkt to extrapoliert, für den ein Wert $\tau(t_0)$ des zeitabhängigen Trocknungsverlaufs unter eine definierte Schwelle $\tau_0$, insbesondere unter eine dem Anwendungsszenario anpassbare Schwelle $\tau_0$, fällt.

In Verfahrensschritt 112 wird überprüft, ob die Berechnungen physikalisch sinnvoll und/oder mathematisch eindeutig sind und/oder konvergieren, sodass belastbare Aussagen aus der Auswertung erhalten werden. Insbesondere kann in einer weiteren Verfahrensschleife eine Wiederholung der vorhergehenden Verfahrensschritte 104-112 erfolgen, wobei in Verfahrensschritt 114 nun weitere Messdaten, insbesondere Messdaten betreffend die sechs zeitabhängigen Feuchteverteilungen $\phi^1(t)$, $\phi^2(t)$, $\phi^3(t)$, $\phi^4(t)$, $\phi^5(t)$, $\phi^6(t)$ für zumindest einen weiteren Zeitpunkt, beispielsweise für einen weiteren Tag, bereitgestellt werden. Ferner ist denkbar, dass in Verfahrensschritt 114 weitere Messdaten betreffend eine zeitabhängige Feuchteverteilung $\phi^i(t)$, die weitere Tiefen d' des Untersuchungsobjekts betrifft, bereitgestellt werden. Ebenfalls können diese weiteren Messdaten in Verfahrensschritt 114a auch unmittelbar am Untersuchungsobjekt 10 gemessen und der Prozessorvorrichtung bereitgestellt werden. Auf diese Weise kann unter Verwendung von Messdaten, die die zeitabhängige Feuchteverteilung $\phi^i(t)$, hier insbesondere die sechs zeitabhängigen Feuchteverteilungen $\phi^1(t)$, $\phi^2(t)$, $\phi^3(t)$, $\phi^4(t)$, $\phi^5(t)$, $\phi^6(t)$, zu oder bis zu einem ersten Zeitpunkt betreffen, das Materialmodell zunächst initial kalibriert werden und mittels der weiteren Messdaten, die die zeitabhängige Feuchteverteilung $\phi^i(t)$, hier die sechs zeitabhängigen Feuchteverteilungen $\phi^1(t)$, $\phi^2(t)$, $\phi^3(t)$, $\phi^4(t)$, $\phi^5(t)$, $\phi^6(t)$, zu weiteren Zeitpunkten betreffen, anschließend die Kalibrierung des Materialmodells, insbesondere parallel zum Trocknungsprozess des Untersuchungsobjekts 10, rekalibriert oder verfeinert werden. Auf diese Weise kann ebenfalls der in Verfahrensschritt 108 ermittelte zeitabhängige Trocknungsverlauf $\tau(t)$ des Untersuchungsobjekts 10 verfeinert bzw. aktualisiert werden. Die Konvergenz oder zunehmende Güte des Materialmodells, insbesondere die Konvergenz des in Verfahrensschritt 108 ermittelten zeitabhängigen Trocknungsverlaufs $\tau(t)$ und/oder des in Verfahrensschritt 110 extrapolierten Trocknungszeitpunkt $t_0$, kann in Verfahrensschritt 112 erneut überprüft werden. Erst wenn eine definierte Qualität des Materialmodells vorliegt - beispielsweise gemessen an einer Konvergenz des extrapolierten Trocknungszeitpunkts $t_0$ - wird die Wiederholungsschleife verlassen.

In diesem Ausführungsbeispiel berücksichtigt das Materialmodell Trocknungsparameter, die den Trocknungsverlauf $\tau(t)$ des Untersuchungsobjekts 10 beeinflussen, und erlaubt somit, diese bei einer Simulation einzubeziehen. Die Trocknungsparameter in diesem Ausführungsbeispiel ermöglichen, zumindest den Einfluss von Lüften (z.B. Luftaustauschrate), Temperaturveränderung (z.B. Wärmezufuhr), Luftfeuchteveränderung, auf den Trocknungsverlauf $\tau(t)$ des Untersuchungsobjekts 10 zu simulieren. In Verfahrensschritt 116 werden diese Trocknungsparameter variiert, wobei optimierte Trocknungsparameter, die in der Simulation zu einem gegenüber den vorliegenden Standardrahmenbedingungen schnelleren oder verkürzten zeitabhängigen Trocknungsverlauf $\tau^{opt}(t)$ des Untersuchungsobjekts 10 führen, berechnet werden.

In Verfahrensschritt 118 werden der in Verfahrensschritt 110 extrapolierte Trocknungszeitpunkt $t_0$ sowie die erhaltenen optimierten Trocknungsparameter als Handlungsanweisung an einen Nutzer des Verfahrens ausgegeben.

In Figur 2 ist eine schematische Perspektivansicht eines beispielhaften Messszenarios unter Verwendung einer beispielhaften Ausführungsform eines erfindungsgemäßen Feuchtemessgeräts 16 dargestellt. Das beispielhaft ausgeführte Feuchtemessgerät 16 weist zumindest einen tiefenauflösenden Feuchtesensor 12 sowie eine Steuervorrichtung (hier nicht näher dargestellt) zur Steuerung des Feuchtemessgeräts 16 auf. Die Steuervorrichtung ist zur Durchführung des erfindungsgemäßen Verfahrens eingerichtet. Die Steuervorrichtung umfasst insbesondere eine Prozessorvorrichtung, eine Speichereinheit und ein in der Speichereinheit gespeichertes Computerprogramm zur Ausführung des erfindungsgemäßen Verfahrens. Das Feuchtemessgerät 16 ist als ein handgehaltenes NMR-Messgerät realisiert, das als Feuchtesensor einen tiefenauflösenden NMR-Sensor 14 aufweist. Der NMR-Feuchtesensor 14 ermöglicht, tiefenaufgelöst Messdaten zu einer Feuchteverteilung $\phi^i$ zumindest bis zu einer maximalen Tiefe $d^{max}$ des Untersuchungsobjekts 16 zu messen, dargestellt in Figur 2 durch variierbare Magnetfelder 18. Die maximale Tiefe $d^{max}$ beträgt in diesem Ausführungsbeispiel 4 cm. Ferner umfasst das Feuchtemessgerät 16 in einem Gehäuse eine Eingabevorrichtung, geeignet zum Ein- und Ausschalten, zum Starten und Konfigurieren eines Messvorgangs sowie zum Eingeben von Arbeitsparametern. Ferner ist in dem Gehäuse eine Ausgabevorrichtung zur Ausgabe von ermittelten Informationen sowie zur Ausgabe von Arbeitsparametern in Form eines Bildschirms vorgesehen. Zur Energieversorgung des Feuchtemessgeräts 16 weist das Feuchtemessgerät 16 eine Aufnahme zur Aufnahme von stromnetzunabhängigen Energiespeichern auf. In Figur 3 sind für das in Figur 2 dargestellte Messszenario ermittelte Kurven dargestellt, die einen zeitabhängigen reduzierten Trocknungsverlauf $\tau^i(t)$ und einen zeitabhängigen Trocknungsverlaufs $\tau(t)$ wiedergeben. Die x-Achse 20 skaliert mit der Zeit, beispielsweise in Tagen, während die y-Achse 22 mit der Feuchtigkeit (beispielsweise in Volumen-%, kg/kg, m3/m3, kg/m3, kg,...) skaliert. Der zeitabhängige reduzierte Trocknungsverlauf $\tau^i(t)$ kann dabei interpretiert werden als die (mittlere) Feuchtigkeit, welche sich während der Trocknung in der Schicht mit Dicke d' befindet. Der Trocknungsverlauf $\tau(t)$ hingegen entspricht dem zeitabhängigen Trocknungsverlauf des gesamten Untersuchungsobjekts 10, d.h. mit vollständiger Tiefe bzw. Dicke.

**Patentansprüche**

1. Computerimplementiertes Verfahren zur Ermittlung eines zeitabhängigen Trocknungsverlaufs $\tau(t)$ eines Untersuchungsobjekts (10), umfassend zumindest die Verfahrensschritte:

    • Bereitstellen von Messdaten betreffend zumindest eine zeitabhängige Feuchteverteilung $\phi^i(t)$ zumindest bis zu einer Tiefe d' des Untersuchungsobjekts (10),
    • Ermitteln eines zeitabhängigen reduzierten Trocknungsverlaufs $\tau^i(t)$ unter Verwendung der zeitabhängigen Feuchteverteilung $\phi^i(t)$, wobei der zeitabhängige reduzierte Trocknungsverlauf $\tau^i(t)$ mittels $\tau^i(t) = \int_0^{d'} \Phi^i(x,\,t)\,dx$ berechnet wird, alternativ durch Mitteln dieses Integrals über $d^i$, wobei die Tiefe d' kleiner ist als eine Gesamtdicke des Untersuchungsobjekts (10),
    • Kalibrieren eines das Untersuchungsobjekt (10) betreffenden Materialmodells unter Verwendung des zeitabhängigen reduzierten Trocknungsverlaufs $\tau^i(t)$,
    • Ermitteln, insbesondere Simulieren, eines zeitabhängigen Trocknungsverlaufs $\tau(t)$ des Untersuchungsobjekts (10) für dessen Gesamtdicke unter Verwendung des kalibrierten Materialmodells, wobei aus dem zeitabhängigen Trocknungsverlauf $\tau(t)$ des Untersuchungsobjekts (10) ein Trocknungszeitpunkt $t_0$ ermittelt wird,

insbesondere extrapoliert wird, für den ein Wert $\tau(t_0)$ des zeitabhängigen Trocknungsverlaufs unter eine Schwelle $\tau_0$ fällt.

2. Computerimplementiertes Verfahren nach einem der vorhergehenden Ansprüche, wobei das Materialmodell zumindest eine Flüssigwasserleitfähigkeit, eine Dampfleitfähigkeit und einen Wasserdampfoberflächenaustausch des Untersuchungsobjekts (10) berücksichtigt.

3. Computerimplementiertes Verfahren nach einem der vorhergehenden Ansprüche, wobei im letzten Verfahrensschritt und/oder in einem weiteren Verfahrensschritt den Trocknungsverlauf $\tau(t)$ des Untersuchungsobjekts (10) beeinflussende Trocknungsparameter simuliert und/oder variiert werden.

4. Computerimplementiertes Verfahren nach Anspruch 3, wobei optimierte Trocknungsparameter, die in der Simulation zu einem schnelleren zeitabhängigen Trocknungsverlauf $\tau^{opt}(t)$ des Untersuchungsobjekts (10) führen, berechnet werden.

5. Computerimplementiertes Verfahren nach Anspruch 4, wobei die optimierten Trocknungsparameter als Handlungsanweisung an einen Nutzer des Verfahrens ausgegeben werden.

6. Computerimplementiertes Verfahren nach einem der vorhergehenden Ansprüche, wobei die Messdaten betreffend die zumindest eine zeitabhängige Feuchteverteilung $\phi^i(t)$ für mindestens sechs verschiedene Zeitpunkte, insbesondere für mindestens sechs verschiedene Tage, bereitgestellt werden.

7. Computerimplementiertes Verfahren nach einem der vorhergehenden Ansprüche, wobei die Messdaten betreffend die zumindest eine zeitabhängige Feuchteverteilung $\phi^i(t)$ für mindestens zwei, insbesondere für mindestens drei, Tiefen d' des Untersuchungsobjekts (10) bereitgestellt werden.

8. Computerimplementiertes Verfahren nach einem der vorhergehenden Ansprüche, wobei die Ermittlung des zeitabhängigen Trocknungsverlaufs $\tau(t)$ des Untersuchungsobjekts (10) unter Verwendung weiterer Messdaten verfeinert wird.

9. Computerimplementiertes Verfahren nach einem der vorhergehenden Ansprüche, wobei die Messdaten unter Verwendung eines tiefenauflösenden Feuchtesensors (12), insbesondere eines tiefenauflösenden NMR-Sensors (14) und/oder eines tiefenauflösenden Feuchtesensorarrays und/oder eines tiefenauflösenden Radars, erfasst und bereitgestellt werden.

10. Feuchtemessgerät (16) mit zumindest einem tiefenauflösenden Feuchtesensor (12) und einer Steuervorrichtung, wobei die Steuervorrichtung zur Durchführung des Verfahrens nach einem der Ansprüche 1-9 eingerichtet ist.

11. Computerprogramm umfassend Befehle, welche bei ihrer Ausführung mittels einer Prozessorvorrichtung das Verfahren nach einem der vorherigen Ansprüche ausführen.

12. Computerlesbares Speichermedium, auf dem ein Computerprogramm nach Anspruch 11 hinterlegt ist.

13. Computervorrichtung, insbesondere Feuchtemessgerät (16), mit zumindest einer Prozessorvorrichtung und einer Speichervorrichtung, wobei auf der Speichervorrichtung Befehle hinterlegt sind, die beim Ausführen dieser durch die Prozessorvorrichtung bewirken, dass die Computervorrichtung ein Verfahren nach einem der Ansprüche 1-9 ausführt.

**Claims**

1. Computer-implemented method for determining a time-dependent drying profile $\tau(t)$ of an examination object (10), comprising at least the following method steps:

   • providing measurement data concerning at least one time-dependent moisture distribution $\phi^i(t)$, at least to a depth $d^i$ of the examination object (10),
   • determining a time-dependent reduced drying profile $\tau^i(t)$ using the time-dependent moisture distribution $\phi^i(t)$, wherein the time-dependent reduced drying profile $\tau^i(t)$ is calculated by means of $\tau^i(t) = \int_0^{d^i} \Phi^i(x, t)\,dx$, alternatively by averaging this integral over $d^i$, wherein the depth $d^i$ is smaller than a total thickness of the examination object (10),
   • calibrating a material model concerning the examination object (10) using the time-dependent reduced drying profile $\tau^i(t)$,
   • determining, in particular simulating, a time-dependent drying profile $\tau(t)$ of the examination object (10) for the total thickness thereof using the calibrated material model, wherein a drying time $t_0$ for which a value $\tau(t_0)$ of the time-dependent drying profile falls below a threshold $\tau_0$ is determined, in particular extrapolated, from the time-dependent drying profile $\tau(t)$ of the examination object (10).

2. Computer-implemented method according to any of the preceding claims, wherein the material model takes into account at least a liquid water conductivity, a vapour conductivity and a water vapour surface exchange of the examination object (10).

3. Computer-implemented method according to any of the preceding claims, wherein drying parameters that influence the drying profile $\tau(t)$ of the examination object (10) are simulated and/or varied in the last method step and/or in a further method step.

4. Computer-implemented method according to Claim 3, wherein optimized drying parameters which lead to a faster time-dependent drying profile $\tau^{opt}(t)$ of the examination object (10) in the simulation are calculated.

5. Computer-implemented method according to Claim 4, wherein the optimized drying parameters are output as an action instruction to a user for the method.

6. Computer-implemented method according to any of the preceding claims, wherein the measurement data concerning the at least one time-dependent moisture distribution $\phi^i(t)$ are provided for at least six different times, in particular for at least six different days.

7. Computer-implemented method according to any of the preceding claims, wherein the measurement data concerning the at least one time-dependent moisture distribution $\phi^i(t)$ are provided for at least two, in particular for at least three, depths $d^i$ of the examination object (10).

8. Computer-implemented method according to any of the preceding claims, wherein the determination of the time-dependent drying profile $\tau(t)$ of the examination object (10) is refined using further measurement data.

9. Computer-implemented method according to any of the preceding claims, wherein the measurement data are acquired and provided using a depth-resolving moisture sensor (12), in particular a depth-resolving NMR sensor (14) and/or a depth-resolving moisture sensor array and/or a depth-resolving radar.

10. Moisture meter (16) comprising at least one depth-resolving moisture sensor (12) and a control device, wherein the control device is configured for carrying out the method according to any of Claims 1-9.

11. Computer program comprising instructions which, when they are executed by means of a processor device, perform the method according to any of the preceding claims.

**12.** Computer-readable storage medium on which a computer program according to Claim 11 is stored.

**13.** Computer device, in particular moisture meter (16), comprising at least one processor device and a storage device, wherein the storage device stores instructions which, when they are executed by the processor device, cause the computer device to perform a method according to any of Claims 1-9.

**Revendications**

**1.** Procédé mis en œuvre par ordinateur pour déterminer un processus de séchage $\tau(t)$ dépendant du temps d'un objet examiné (10), comprenant au moins les étapes de procédé suivantes :

> * fourniture de données de mesure concernant au moins une distribution d'humidité $\phi^i(t)$ dépendante du temps au moins jusqu'à une profondeur $d^i$ de l'objet examiné (10),
> * détermination d'un processus de séchage $\tau^i(t)$ réduit dépendant du temps en utilisant la distribution d'humidité $\phi^i(t)$ dépendante du temps, le processus de séchage $\tau^i(t)$ dépendant du temps étant calculé au moyen de
>
> $$\tau^i(t) = \int_0^{d^i} \Phi^i(x,\ t)\,dx$$
>
> , en variante par un calcul de la moyenne de cette intégrale sur $d^i$, la profondeur $d^i$ étant inférieure à une épaisseur totale de l'objet examiné (10),
> * étalonnage d'un modèle de matériel concernant l'objet examiné (10) en utilisant le processus de séchage $\tau^i(t)$ réduit dépendant du temps,
> * détermination, notamment simulation, d'un processus de séchage $\tau(t)$ dépendant du temps de l'objet examiné (10) pour son épaisseur totale en utilisant le modèle de matériel étalonné,
>
> un instant de séchage $t_0$ étant déterminé, notamment extrapolé, à partir du processus de séchage $\tau(t)$ dépendant du temps de l'objet examiné (10), pour lequel une valeur $\tau(t_0)$ du processus de séchage dépendant du temps chute au-dessous d'un seuil $\tau_0$.

**2.** Procédé mis en œuvre par ordinateur selon l'une des revendications précédentes, le modèle de matériel tenant compte d'au moins une conductivité de l'eau liquide, d'une conductivité de la vapeur et d'un échange en surface de vapeur de l'objet examiné (10).

**3.** Procédé mis en œuvre par ordinateur selon l'une des revendications précédentes, des paramètres de séchage qui influencent le processus de séchage $\tau(t)$ de l'objet examiné (10) étant simulés et/ou mis à varier dans la dernière étape du procédé et/ou dans une étape supplémentaire du procédé.

**4.** Procédé mis en œuvre par ordinateur selon la revendication 3, des paramètres de séchage optimisés qui, dans la simulation, conduisent à un processus de séchage $\tau^{opt}(t)$ dépendant du temps plus rapide de l'objet examiné (10), étant calculés.

**5.** Procédé mis en œuvre par ordinateur selon la revendication 4, les paramètres de séchage optimisés étant délivrés à un utilisateur du procédé sous la forme d'une instruction de manipulation.

**6.** Procédé mis en œuvre par ordinateur selon l'une des revendications précédentes, les données de mesure concernant l'au moins une distribution d'humidité $\phi^i(t)$ dépendante du temps étant mises à disposition pour au moins six instants différents, notamment pour au moins six jours différents.

**7.** Procédé mis en œuvre par ordinateur selon l'une des revendications précédentes, les données de mesure concernant l'au moins une distribution d'humidité $\phi^i(t)$ dépendante du temps étant mises à disposition pour au moins deux, notamment pour au moins trois profondeurs $d^i$ de l'objet examiné (10).

**8.** Procédé mis en œuvre par ordinateur selon l'une des revendications précédentes, la détermination du processus de séchage $\tau(t)$ dépendant du temps de l'objet examiné (10) étant affinée en utilisant des données de mesure supplémentaires.

**9.** Procédé mis en œuvre par ordinateur selon l'une des revendications précédentes, les données de mesure étant acquises et mises à disposition en utilisant un capteur d'humidité (12) à résolution de profondeur, notamment un capteur NMR (14) à résolution de profondeur et/ou un réseau de capteurs d'humidité à résolution de profondeur et/ou un radar à résolution de profondeur.

**10.** Appareil de mesure d'humidité (16) comprenant au moins un capteur d'humidité (12) à résolution de profondeur et un dispositif de commande, le dispositif de commande étant conçu pour mettre en œuvre le procédé selon l'une des revendications précédentes.

**11.** Programme informatique comprenant des commandes qui, lors de leur exécution au moyen d'un dispositif à processeur, mettent en œuvre le procédé selon l'une des revendications précédentes.

**12.** Support d'enregistrement lisible par ordinateur, sur lequel est stocké un programme informatique selon la revendication 11.

**13.** Dispositif informatique, notamment appareil de mesure d'humidité (16), comprenant au moins un dispositif à processeur et un dispositif de mémorisation, des instructions étant stockées sur le dispositif de mémorisation qui, lorsqu'elles sont exécutées par le dispositif à processeur, ont pour effet que le dispositif informatique met en œuvre un procédé selon l'une des revendications 1 à 9.

Figur 1

Figur 2

Figur 3

**EP 3 963 314 B1**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014218375 A1

**In der Beschreibung aufgeführte Nicht-Patentliteratur**

- **THOMAS et al.** Moisture transport in pine wood during one-sided heating studied by NMR. *Eperimental Thermal and Fluid Science,* 04. August 2018, vol. 99, 259-271
- **PROIETTI N. et al.** Evolution of physicochemical properties of pear during drying by conventional tehcniques, portable-NMR, and modelling. *Journal of Food Engineering,* 28. Februar 2018, vol. 230, 82-98

- **BURDINE.** *Relative Permeability Calculations From Pore Size Distribution Data,* 1953
- **MUALEM.** *A new model for predicting the hydraulic conductivity of unsaturated porous media,* 1976
- **P. HÄUPL ; J. GRUNEWALD ; H. FECHNER ; H. STOPP.** *Coupled heat air and moisture transfer in building structures*

15